(19) Europäisches Patentamt
European Patent Office
Office européen des brevets

(11) Publication number: **0 098 137**
**A2**

(12) **EUROPEAN PATENT APPLICATION**

(21) Application number: 83303695.7

(22) Date of filing: 27.06.83

(51) Int. Cl.³: C 12 P 7/26
C 12 P 7/02, C 12 P 17/02
C 12 N 9/02

(30) Priority: 28.06.82 US 392609
28.06.82 US 392610
28.06.82 US 392612

(43) Date of publication of application:
11.01.84 Bulletin 84/2

(84) Designated Contracting States:
DE FR GB

(71) Applicant: Exxon Research and Engineering Company
P.O.Box 390 180 Park Avenue
Florham Park New Jersey 07932(US)

(72) Inventor: Hou, Ching-Tsang
14 Pendleton Place
Edison New Jersey(US)

(72) Inventor: Laskin, Allen I.
RD 3/Box 392T
Somerset New Jersey(US)

(72) Inventor: Patel, Ramesh Narsibhai
15 Mockingbird Road
Edison New Jersey(US)

(74) Representative: Field, Roger Norton et al,
ESSO Engineering (Europe) Ltd. Patents & Licences
Apex Tower High Street
New Malden Surrey KT3 4DJ(GB)

(54) **A microbiological process for the oxidation of alkanes, vinyl compounds and secondary alcohols.**

(57) A substrate selected from alkanes, saturated alicyclic or aromatic hydrocarbons, alkenes, dienes, vinyl aromatic compounds or secondary alcohols is oxidized to the corresponding oxidation product(s) by contacting it in a reaction medium under aerobic conditions with microbial cells derived from a selected microorganism or a genetically engineered derivative or natural mutant thereof or an enzyme preparation prepared from the cells, wherein the microorganism has been previously grown under aerobic conditions in a nutrient medium containing a $C_2$-$C_6$ alkane or, if the substrate is a secondary alcohol, also a $C_2$-$C_6$ alkyl radical donating compound such as a linear alcohol or alkylamine.

EP 0 098 137 A2

The present invention relates to the oxidation of alkanes and other saturated hydrocarbons, alkenes, dienes, vinyl aromatic compounds and secondary alcohols through the action of oxygen and microbial cells of microorganisms, or enzyme preparations derived therefrom, which utilize $C_2$-$C_6$ alkanes as the major carbon and energy source. This invention also relates to oxidation of secondary alcohols via action of oxygen and microbial cells of microorganisms, or enzyme preparations derived therefrom, which utilize $C_2$-$C_6$ alkyl radical donating compounds such as $C_2$-$C_6$ linear primary alcohols and $C_2$-$C_6$ alkylamines.

Methane is not the only hydrocarbon substrate on which microorganisms can be grown. Depending on the particular bacterial strain employed, gaseous and liquid hydrocarbons have been known to be effective as growth substrates. The genera of microorganisms known to utilize hydrocarbons other than methane as carbon and energy source broadly include, e.g., *Achromobacter*, *Acinetobacter*, *Pseudomonas*, *Nocardia*, *Bacillus*, *Mycobacterium*, *Corynebacterium*, *Brevibacterium*, *Candida*, *Arthrobacter*, *Streptomycetes*, *Flavobacterium*, and various filamentous fungi. It is often difficult, however, to predict, for any given combination of microorganism strain and hydrocarbon growth substrate, the precise behavior of the strain in a culture medium.

U.S. Patent 3,344,037 discloses a method for enzymatically oxidizing an oxidized hydrocarbon such as an alcohol or aldehyde to an oxidation product with the same number of carbon atoms using a microorganism grown on a hydrocarbon corresponding substantially to the oxidizable hydrocarbon in number of carbon atoms.

Preferably, the microorganism is chosen from the genera of *Achromobacter*, *Pseudomonas*, *Nocardia*, *Bacillus* and *Mycobacterium*, most preferably *Achromobacter* grown on decane.

J. W. Foster, *Ant. v. Leeu.*, *J. Microbiol. & Ser.*, *28*, 241 (1962) provides a review of organisms grown on hydrocarbons, and more recently, J. J. Perry, *Microbiol.* *Rev.*, *43*, 59 (1979) reviews studies of cooxidation of substrates using microorganisms grown on hydrocarbons. In cooxidations, the non-growth substrates are oxidized when present as co-substrates in a growth medium containing the growth substrate. Cooxidation is thus not applicable to oxidations using resting cells.

A.S. Kester, PhD Diss., Univ. of Texas (1961) tested twenty-three microorganisms, representing the genera *Nocardia*, *Brevibacterium*, *Pseudomonas*, *Alkaligenes* or *Achromobacter*, *Corynebacterium*, *Mycobacterium*, *Streptomyces* and *Fusarium*, for their capacity to grow on $C_1$-$C_{18}$ hydrocarbons, the capacities varying with the particular microorganism tested.

A process is herein disclosed for oxidizing a substrate selected from alkanes, saturated alicyclic or aromatic hydrocarbons, alkenes, dienes, vinyl aromatic compounds or secondary alcohols to the corresponding oxidation product or products comprising contacting the substrate in a reaction medium under aerobic conditions with microbial cells derived from a microorganism, a genetically engineered derivative or natural mutant of the microorganism or an enzyme preparation prepared from the cells (wherein the microorganism strains are selected from those strains described hereinafter),

which microorganism, derivative, mutant or preparation exhibits oxygenase or dehydrogenase enzyme activity, until the corresponding product is produced in at least isolatable amounts, wherein the microorganism has been previously grown under aerobic conditions in a nutrient medium containing a compound which is a $C_2$-$C_6$ alkane or, if the substrate is an alcohol, the compound may additionally be a $C_2$-$C_6$ alkyl radical donating compound.

In a preferred embodiment the substrate is a $C_2$-$C_6$ alkane, cyclohexane, benzene, a $C_2$-$C_7$ alkene, butadiene, styrene or a $C_3$-$C_6$ secondary alcohol for alkane-grown microorganisms and a $C_3$-$C_5$ secondary alcohol for microorganisms grown on alkyl radical donating compounds. Most preferably, the substrate is propane, n-butane, cyclohexane, benzene, ethylene, propylene, 2-propanol or 2-butanol. The $C_2$-$C_6$ alkane on which the microorganisms may be grown is preferably a $C_2$-$C_4$ alkane, e.g., ethane, propane or butane, and the $C_2$-$C_6$ alkyl radical donating compound is a $C_2$-$C_4$ compound, e.g., ethanol, propanol, butanol, ethylamine, propylamine or butylamine.

In another preferred embodiment the microbial cells are resting cells, and more preferably are in the form of a resting cell suspension. In yet another preferred embodiment the enzyme preparation prepared from the cells is a cell-free soluble fraction.

The term "microorganism" is used herein in its broadest sense to include all of the microorganism strains identified below, which are generally bacterial strains.

The expression "genetically engineered derivatives of a microorganism" is used herein in the sense recognized by those skilled in the art, and includes artificial mutants and recombinant DNA-produced microorganisms.

The term "enzyme preparation" is used to refer to any composition of matter that exhibits the desired dehydrogenase enzymatic activity. The term is used to refer, for example, to live whole cells, dried cells, cell-free particulate and soluble fractions, cell extracts, and refined and concentrated preparations derived from the cells. Enzyme preparations may be either in dry or liquid form. The term also includes the immobilized form of the enzyme, e.g., the whole cells of the $C_2$-$C_6$-alkane or alkyl radical donating compound-grown microorganisms or enzyme extracts which are immobilized or bound to an insoluble matrix by covalent chemical linkages, absorption and entrapment of the enzyme within a gel lattice having pores sufficiently large to allow the molecules of the substrate and the product to pass freely, but sufficiently small to retain the enzyme. The term "enzyme preparation" also includes enzymes retained within hollow fiber membranes, e.g., as disclosed by Rony, Biotechnology and Bioengineering (1971).

The term "$C_2$-$C_6$ alkyl radical donating compound" refers to a $C_2$-$C_6$ alkyl compound which will donate ethyl, propyl, butyl, pentyl or hexyl radicals, such as ethanol, propanol, butanol, pentanol, hexanol, ethylamine, propylamine, butylamine, ethyl formate, butyl carbonate, etc. Such compounds can also be characterized as growth substrates which are capable of inducing dehydrogenase enzyme activity in the microorganism strains described below.

The term "resting cells" refers to cells which are not maintained in a growth or nutrient medium (i.e., containing a carbon and nitrogen source) so that they are not being cultured where they can actively grow and multiply while the oxidation is being carried out. Typically, resting cells are prepared by harvesting the cultured cells, washing them with buffer and suspending them in the buffer so as to obtain a resting cell suspension for use in the oxidation. Thus, the nutrient growth medium is not present during the conversion. However, various materials which assist the enzymatic conversion such as, e.g., cofactors, salts, water, promoters, etc. may be present during the conversion.

As one embodiment of the present invention, it has been discovered that methyl ketones and alcohols are produced by contacting the corresponding alkane or saturated alicyclic or aromatic hydrocarbon under aerobic conditions with microbial cells derived from the microorganism strains given below (or equivalents of the cells such as genetically engineered derivatives of the microorganisms or enzyme preparations prepared therefrom), wherein the microorganisms have been previously grown under aerobic conditions in a nutrient medium containing a $C_2$-$C_6$ alkane growth substrate, such as ethane, propane, butane, pentane, 2-methylbutane, hexane, etc., preferably a $C_2$-$C_4$ alkane, and most preferably propane. Preferred microorganism strains for this embodiment include Pseudomonas aeruginosa strains, particularly those grown on $C_2$-$C_4$ alkanes.

The alkane oxidative substrate herein may be selected from such alkanes as, for example, propane, n-butane, isobutane, n-pentane, isopentane, neopentane, n-hexane, 2,2-dimethylbutane, 2,3-dimethylbutane, etc. Most preferably, the alkane is propane or n-butane. The

saturated alicyclic (i.e., cycloaliphatic) or aromatic hydrocarbon herein includes cycloalkanes such as cyclopropane, cyclobutane, cyclopentane and cyclohexane, and aromatic and aryl alkyl compounds such as benzene and toluene. The preferred such compounds herein are cyclohexane and benzene.

As another embodiment of the present invention it has been discovered that 1,2-epoxides are produced by contacting the corresponding alkene, diene or vinyl aromatic compound under aerobic conditions with resting microbial cells derived from microorganism strains given below (or equivalents of the cells such as genetically engineered derivatives of the microorganisms or enzyme preparations prepared therefrom), wherein the microorganisms have been previously grown under aerobic conditions in a nutrient medium containing a $C_2$-$C_6$ alkane such as ethane, propane, butane, pentane, 2-methylbutane, hexane, etc. Preferably, the growth substrate is a $C_2$-$C_4$ alkane, and most preferably propane. Preferred microorganism strains herein include the Pseudomonas aeruginosa strains, particularly when a $C_2$-$C_4$ alkane is employed.

The olefin substrate herein may be selected from, e.g., ethylene, propylene, 1-butene, cis or trans-but-2-ene, isobutene, 1-pentene, 2-pentene, butadiene, isoprene, styrene, etc. Preferably, the olefin substrate is a $C_2$-$C_7$ alkene (more preferably a $C_2$-$C_6$ alkene), butadiene or styrene. Most preferably, the alkene is ethylene or propylene.

As a third embodiment of the present invention, it has been discovered that methyl ketones are produced by contacting the corresponding secondary alcohol, preferably a $C_3$-$C_6$ secondary alcohol, under aerobic

conditions with microbial cells derived from the microorganisms given below (or equivalents of the cells such as genetically engineered derivatives of the microorganisms or enzyme preparations prepared therefrom), wherein the microorganisms have been previously grown under aerobic conditions in a nutrient medium containing a $C_2$-$C_6$ alkane, preferably a $C_2$-$C_4$ alkane. Examples of $C_2$-$C_6$ alkanes are ethane, propane, butane, isobutane, pentane, 2-methyl-butane, hexane, etc. Most preferably the alkane herein is propane. Preferred microorganism strains herein include the Pseudomonas aeruginosa strains, particularly when a $C_2$-$C_4$ alkane is employed.

As a fourth embodiment of the invention it has been discovered that methyl ketones are produced by contacting the corresponding secondary alcohol, preferably $C_3$-$C_5$ secondary alcohol, under aerobic conditions with microbial cells derived from microorganisms (or equivalents thereof), wherein the microorganisms have been previously grown aerobically in a nutrient medium containing a $C_2$-$C_6$ alkyl radical donating compound, preferably $C_2$-$C_4$ linear primary alcohols or $C_2$-$C_4$ alkylamines.

The secondary alcohol which is to be oxidized may be selected from such alcohols as, for example, 2-propanol, 2-butanol, 2-pentanol, 3-pentanol, 2-hexanol and 3-hexanol. Preferably, the alcohol is a linear secondary alcohol and, most preferably, 2-propanol or 2-butanol.

The present invention includes the following features:

o Cell suspensions of the microorganism strains identified below oxidize $C_3$-$C_6$ alkanes to

their corresponding secondary alcohols and methyl ketones. Among the n-alkanes, propane and butane are oxidized at the highest rate.

o Cell-free soluble extracts derived from propane-grown Pseudomonas fluorescens NRRL B-1244 oxidize $C_2$-$C_6$ alkanes, cyclohexane and toluene to their corresponding alcohols.

o The strain tested exhibits an optimum oxidation temperature of 35°C and an optimum oxidation pH of about 7.5 for oxidation of propane and butane.

o Cell suspensions of at least three microorganism strains identified below generally oxidize cyclohexane and benzene to their corresponding alcohols. Butane-grown Brevibacterium fuscum ATCC 15993 is found to oxidize toluene to benzylalcohol.

o Resting cell suspensions of the microorganism strains identified below oxidize (epoxidize) alkenes, dienes and vinyl aromatic compounds to their corresponding 1,2-epoxides. The product 1,2-epoxides accumulate extracellularly and are produced enzymatically. Among the alkenes, ethylene and propylene are oxidized at the highest rate.

o Propanol-grown cells of the strains herein do not convert the olefins to 1,2-epoxides.

o The strains tested have an optimum temperature of 35°C and an optimum pH of between 6.0 and 7.0.

o The epoxidation may be carried out in the presence of an electron carrier or other biochemical species which drives the epoxidation, such as NADH.

Such electron carrier or other biochemical species can be regenerated,, e.g., by adding ethanol to the reaction medium.

o Cell suspensions of the $C_2$-$C_6$-alkane-grown microorganism strains identified below oxidize $C_3$-$C_6$ secondary alcohols to their corresponding methyl ketones. Cell suspensions of the $C_2$-$C_6$-alkyl radical donating compound-grown microorganism strains oxidize $C_3$-$C_6$ secondary alcohols to their corresponding methyl ketones. Among the $C_3$-$C_6$ secondary alcohols, 2-propanol and 2-butanol are oxidized at the highest rate.

o The strain tested exhibits an optimum oxidation temperature of 35°C and an optimum oxidation pH of about 8.0 to 9.0 for oxidation of 2-propanol and 2-butanol.

The microorganism strains employed in the present invention are all known strains and are classified according to the classification system described in Bergey's Manual of Determinative Bacteriology, Robert S. Breed et al., eds., 8th ed. (Baltimore: Williams & Wilkins Co., 1974). Subcultures of each strain were deposited either with the depository of the American Type Culture Collection (ATCC) in Rockville, Maryland 20852 or with the depository of the U.S. Department of Agriculture, Northern Regional Research Laboratory (NRRL) in Peoria, Illinois 61604 and are now publicly available. Each subculture received from the depository the individual ATCC or NRRL strain designation as indicated below. It will be noted that some of the microorganisms have two designations as indicated in the ATCC Catalog of Strains I, 15th ed., 1982. The following organism strains are useful in any of the oxidation reactions described above:

| | Culture | ATCC or NRRL Designation |
|---|---|---|
| 1. | *Rhodococcus rhodochrous* (*Arthrobacter* sp.) | ATCC 19140 |
| 2. | *Pseudomonas aeruginosa* (*Alcaligenes* sp.) | ATCC 15525 |
| 3. | *Arthrobacter petroleophagus* | ATCC 21494 |
| 4. | *Arthrobacter simplex* | ATCC 21032 |
| 5. | *Pseudomonas aeruginosa* (*Brevibacterium insectiphilum*) | ATCC 15528 |
| 6. | *Brevibacterium* sp. | ATCC 14649 |
| 7. | *Brevibacterium fuscum* | ATCC 15993 |
| 8. | *Alcaligenes eutrophus* (*Hydrogenomonas eutropha*) | ATCC 17697 |
| 9. | *Mycobacterium album* | ATCC 29676 |
| 10. | *Rhodococcus rhodochrous* (*Mycobacterium rhodochrous*) | ATCC 29670 |
| 11. | *Rhodococcus rhodochrous* (*Mycobacterium rhodochrous*) | ATCC 29672 |
| 12. | *Rhodococcus sp.* (*Nocardia neoopaca*) | ATCC 21499 |
| 13. | *Pseudomonas crucurae* | NRRL B-1021 |
| 14. | *Pseudomonas fluorescens* | NRRL B-1244 |
| 15. | *Pseudomonas cepacia* (*Pseudomonas multivorans*) | ATCC 17616 |
| 16. | *Pseudomonas putida* Type A | ATCC 17453 |
| 17. | *Pseudomonas aeruginosa* (*Pseudomonas ligustri*) | ATCC 15522 |

If the substrate is an alkene, diene or vinyl aromatic compound, the microorganism strains may additionally be selected from the group consisting of:

*Mycobacterium paraffinicum* ATCC 12670
*Rhodococcus rhodochrous* (*Mycobacterium rhodochrous*) ATCC 184

If the substrate is a secondary alcohol, the microorganism strains may additionally be selected from the group consisting of:

*Mycobacterium paraffinicum* ATCC 12670
*Rhodococcus rhodochrous* (*Nocardia paraffinica*) ATCC 21198

If the substrate is an alkane or saturated alicyclic or aromatic hydrocarbon, the microorganism strain may additionally be:

*Rhodococcus rhodochrous* (*Nocardia paraffinica*) ATCC 21198

The morphological and taxonomical characteristics of the above strains, as well as literature references or patents where the strains are described, are indicated as follows:

*Rhodococcus rhodochrous* (*Arthrobacter* sp.) ATCC 19140 (M. Goldberg et al., *Can. J. Microbiol.*, *3*, 329 (1957)) The organisms are Gram-negative rods, usually very short and plump in logarithmic phase, approaching coccus shape in stationary phase, predominantly in pairs and short chains, immotile, catalase positive. Grow aerobically on nutrient broth, succinate, glucose, aliphatic hydrocarbons and n-paraffins. Do not grow on methane.

*Pseudomonas aeruginosa* (*Alcaligenes* sp.) ATCC 15525 (U.S. Pat. No. 3,308,035, Re. 26,502 and U.S. Pat. No. 3,301,766 to Esso Research and Engineering Co.) The organisms are Gram-negative small rods, immotile. Ferment glucose. Grow aerobically on $C_2$-$C_{30}$ aliphatic hydrocarbons, e.g., $C_2$-$C_{30}$ n-paraffins and $C_6$-$C_{30}$ olefins.

*Arthrobacter petroleophagus* ATCC 21494 (Strain No. 2-15) (U.S. Pat. No. 3,762,997 to Nippon Oil Co. Ltd.) The organisms are Gram-positive or negative, immotile rods. Grow aerobically on $C_2$-$C_5$ and $C_{11}$-$C_{18}$ n-paraffins, glucose, citrate and glycerol. Do not grow on methane.

Arthrobacter simplex ATCC 21032 (B-129 strain) (U.S. Pat. No. 3,622,465 to Allied Chemical Corp.) The organisms are Gram-negative cells in both rod and coccus forms, motile, non-spore forming. Grow aerobically on $C_3$-$C_{18}$ n-paraffins. Do not grow on methane.

Pseudomonas aeruginosa (Brevibacterium insectiphilum) ATCC 15528 (U.S. Pat. No. 3,308,035 and Re. 26,502 to Esso Research and Engineering Co.) The organisms are Grampositive small rods, immotile. Ferment glucose. Grow aerobically on $C_2$-$C_{30}$ aliphatic hydrocarbons, e.g., $C_2$-$C_{30}$ n-paraffins and $C_6$-$C_{30}$ olefins.

Brevibacterium sp. ATCC 14649 (U.S. Pat. No. 3,239,394 to Merck & Co. Inc.) The organisms are Gram-positive short rods. Grow on $C_2$-$C_{10}$ alkanes and alcohols and on nutrient agar. Do not grow on methane.

Brevibacterium fuscum ATCC 15993 (N. Saito et al., Agr. Biol. Chem., 28, 48 (1964) and N. Saito, Biochem. J., 57, 7 (1965)) The organisms are Gram-negative, short, unbranched rods, reproducing by simple cell division and placed in the family Brevibacteriaceae, motile. Produce brownish, yellowish or orange pigments. Grow aerobically on nutrient broth, glucose, succinate, aliphatic hydrocarbons and paraffins. Do not grow on methane.

Alcaligenes eutrophus (Hydrogenomonas eutropha) ATCC 17697 (Intern. J. Syst. Bacteriol., 19, 385 (1969) (type strain), B.F. Johnson et al., J. Bacteriol., 107, 468 (1971) and D. H. Davis et al., Arch. Mikrobiol., 70, 2 (1970)) The organisms are Gram-negative unicellular rods, motile with peritrichous flagella. Non-spore forming. Colonies are opaque, white or cream colored. Grow aerobically on nutrient broth, glucose, succinate,

aliphatic hydrocarbons and paraffins. Do not grow on methane.

Mycobacterium album ATCC 29676 (J. J. Perry et al., J. Bacteriol., 112, 513 (1972), J. Gen. Microbiol., 82, 163 (1974)) The organisms are not readily stainable by Gram's method but are usually considered Gram-positive. Immotile, slightly curved or straight rods, sometimes branching, filamentous or mycelin-like growth may occur. Acid-alcohol fast at some stage of growth. Grow aerobically on paraffins and aliphatic hydrocarbons. Do not grow on methane.

Rhodococcus rhodochrous (Mycobacterium rhodochrous) ATCC 29670 (J.J. Perry et al., J. Bacteriol., 112, 513 (1972)) The organisms are not readily stainable by Gram's method but usually are considered Gram-positive. Immotile, short, plump rods, slightly curved with rounded or occasionally thickened ends, branching rare but occasionally Y-shaped cells observed. Acid-alcohol fast. Yellow to orange pigments. Grow aerobically on paraffins and aliphatic hydrocarbons. Do not grow on methane.

Rhodococcus rhodochrous (Mycobacterium rhodochrous) ATCC 29672 (J. J. Perry et al., J. Bacteriol., 94, 1919 (1967), J. Bacteriol., 96, 318 (1968), Arch. Mikro., 91, 87 (1973), J. Gen. Microbiol., 82, 163 (1974), and J. Bacteriol., 118, 394 (1974)) Same characteristics as ATCC 29670.

Rhodococcus sp. (Nocardia neoopaca) ATCC 21499 (Strain 2-53) (U.S. Pat. No. 3,762,997 to Nippon Oil Co. Ltd.) The organisms are Gram-positive rods or filaments, immotile. Grow aerobically on $C_2$-$C_4$ and $C_{11}$-$C_{18}$ n-paraffins, glucose, gluconate, citrate and succinate. Do not grow on methane.

Pseudomonas crucurae NRRL B-1021 (Bergey's Manual of Determinative Bacteriology, 8th ed., 1974, supra) The organisms are Gram-negative, aerobic rods. Motile by polar monotrichous flagella. Do not produce fluorescent pigment. Grow aerobically on $C_2$-$C_{10}$ alkanes, $C_2$-$C_{10}$ primary alcohols, butylamine, and nutrient agar. Do not grow on methane.

Pseudomonas fluorescens NRRL B-1244 (Bergey's Manual of Determinative Bacteriology, 8th ed., 1974, supra)) The organisms are Gram-negative, aerobic rods, motile. Produce fluorescent pigment. Hydrolyze gelatin. Nonspore forming. Produce small yellow colonies on mineral salt plates in the presence of $C_2$-$C_{10}$ alkanes and primary alcohols. Also grow on nutrient media. Do not grow on methane.

Pseudomonas cepacia (Pseudomonas multivorans) ATCC 17616 (R.Y. Stanier et al., J. Gen. Microbiol., 43, 159 (1966) and R. W. Ballard et al., J. Gen. Microbiol., 60, 199 (1970)) The organisms are Gram-negative, unicellular rods, motile with polar multitrichous flagella. Hydrolyze gelatin. Produce phenazine pigment. Non-spore forming. Do not hydrolyze starch. Grow aerobically on succinate, glucose, nutrient broth, paraffins, and aliphatic hydrocarbons, including $C_2$-$C_4$ alcohols and butylamine. Do not grow on methane.

Pseudomonas putida Type A ATCC 17453 (R. Y. Stanier et al., J. Gen. Microbiol., 43, 159 (1966)) The organisms are Gram-negative unicellular rods, motile with polar multitrichous flagella. Do not hydrolyze gelatin or denitrify. Produce diffusible fluorescent pigment. Non-spore forming. Grow aerobically on succinate, nutrient broth, glucose, paraffins and aliphatic hydrocarbons, including $C_2$-$C_4$ alcohols and butylamine. Do not grow on methane.

*Pseudomonas aeruginosa* (*Pseudomonas ligustri*) ATCC 15522 (U.S. Pat. No. 3,308,035, Re. 26,502 and U.S. Pat. No. 3,301,766 to Esso Research and Engineering Co.) The organisms are Gram-negative small rods, motile. Ferment starch and glucose. Produce fluorescent and phenazine pigment. Non-spore forming. Grow aerobically on $C_2$-$C_{30}$ aliphatic hydrocarbons, e.g., $C_2$-$C_{30}$ n-paraffins and $C_6$-$C_{30}$ olefins.

*Mycobacterium paraffinicum* ATCC 12670 (J. B. Davis et al., *App. Microbiol.*, 4, 310 (1956) to Magnolia Petroleum Co.) The organisms are Gram-positive slender rods, immotile and acid alcohol fast at some stage of growth. Irish lipid content in cells and cell walls. Colonies are regularly or variably yellow or orange usually due to carotinoid pigments. Grow aerobically on paraffinic hydrocarbons such as $C_2$-$C_{10}$ n-alkanes, aliphatic hydrocarbons, ethanol and acetate. Do not grow on methane.

*Rhodococcus rhodochrous* (*Mycobacterium rhodochrous*) ATCC 184 (R.S. Breed, *J. Bacteriol.*, 73, 15 (1957)) Same characteristics as ATCC 29670.

*Rhodococcus rhodochrous* (*Nocardia paraffinica*) ATCC 21198 (U.S. Pat. No. 3,751,337 to Kyowa Hakko Kogyo Co. Ltd.) The organisms are Gram-positive rods or cells, immotile. Ferment sugars. Grow aerobically on $C_2$-$C_4$ and $C_{12}$-$C_{17}$ n-alkanes. Do not grow on methane.

It will be understood that genetically engineered derivatives of these microorganisms may also be used in producing microbial cells and enzyme preparations derived from these cells.

The maintenance of the cultures of these

strains should be carefully controlled. The preferred means for maintaining the cultures is described below.

## MAINTENANCE OF CULTURES

The organisms are preferably subcultured every two weeks on mineral salts agar plates which contain medium described in Example 1.

These plates should be incubated in glass dessicators which have lids with an airtight seal and external sleeves with a tooled hose connection. Dessicators are to be evacuated and filled typically with a gas mixture of $C_2$-$C_4$ alkane and air (1:1 v/v). Incubation should be at 30°C. Cultures will survive in these dessicators for three months at 4°C. However, frequent transfer of cultures is preferred.

In commercial processes for the propagation of microorganisms, it is generally necessary to proceed by stages. These stages may be few or many, depending on the nature of the process and the characteristics of the microorganisms. Ordinarily, propagation is started by inoculating cells from a slant of a culture into a presterilized nutrient medium usually contained in a flask. In the flask, growth of the microorganisms is encouraged by various means, e.g., shaking for thorough aeration, and maintenance of suitable temperature. This step or stage is repeated one or more times in flasks or vessels containing the same or larger volumes of nutrient medium. These stages may be conveniently referred to as culture development stages. The microorganisms with or without accompanying culture medium, from the last development stage, are introduced or inoculated into a large-scale fermentor to produce commercial quantities of the microorganisms or enzymes derived therefrom.

Reasons for growing the microorganisms in stages are manifold, but are primarily dependent upon the conditions necessary for the growth of the microorganisms and/or the production of enzymes therefrom. These include stability of the microorganisms, proper nutrients, pH, osmotic relationships, degree of aeration, temperature and the maintenance of pure culture conditions during fermentation. For instance, to obtain maximum yields of the microbial cells, the conditions of fermentation in the final stage may have to be changed somewhat from those practised to obtain growth of the microorganisms in the culture development stages. Maintaining the purity of the medium, also, is an extremely important consideration, especially where the fermentation is performed under aerobic conditions as in the case of the $C_2$-$C_6$ alkyl compound utilizing microorganisms of this invention. If the fermentation is initially started in a large fermentor, a relatively long period of time will be needed to achieve an appreciable yield of microorganisms and/or oxygenase or dehydrogenase enzymes therefrom. This, of course, enhances the possibility of contamination of the medium and mutation of the microorganisms.

The culture media used for growing the microorganisms and inducing the oxidative enzyme system will be comprised of inorganic salts of phosphate, sulfates and nitrates as well as oxygen and a source of $C_2$-$C_6$ alkyl compounds. The fermentation will generally be conducted at temperatures ranging from 5° to about 50°C, preferably at temperatures ranging from about 25° to about 45°C. The pH of the culture medium should be controlled at a pH ranging from about 4 to 9 and preferably from about 5.5 to 8.5 and more preferably from 6.0 to 7.5. The fermentation may be conducted at atmospheric pressures, although higher pressures up to about 5 atmospheres and higher may be employed.

Typically, to grow the microorganisms and to induce the oxygenase and dehydrogenase enzyme activity, the microorganisms are inoculated into the medium which contains the enzyme-inducing growth and energy substrate (e.g., ethane, butane, propane, ethanol, propanol, butanol. ethylamine, propylamine, butylamine, etc.) and oxygen. For continuous flow culture the microorganisms may be grown in any suitably adapted fermentation vessel, for example, a stirred baffled fermentor or sparged tower fermentor, which is provided either with internal cooling or an external recycle cooling loop. Fresh medium may be continuously pumped into the culture at rates equivalent to 0.02 to 1 culture volume per hour and the culture may be removed at a rate such that the volume of culture remains constant. The inducer-growth substrate-oxygen mixture and possibly carbon dioxide or other gases is contacted with the medium preferably by bubbling continuously through a sparger at the base of the vessel. The source of oxygen for the culture may be air, oxygen or oxygen-enriched air. Spent gas may be removed from the head of the vessel. The spent gas may be recycled either through an external loop or internally by means of a gas inducer impeller. The gas flows and recycle should be arranged to give maximum growth of microorganism and maximum utilization of the inducing-growth substrate.

The microbial cells may be harvested from the growth medium by any of the standard techniques commonly used, for example, flocculation, sedimentation and/or precipitation, followed by centrifugation and/or filtration. The biomass may also be dried, e.g., by freeze or spray drying and may be used in this form for further use in the oxidative conversion process. In the case of the alcohol dehydrogenase enzyme system one may conveniently use the enzyme in the form of the soluble

extract (which may be optionally immobilized onto an inert carrier).

To put the invention to practice, the oxygenase and alcohol dehydrogenase enzyme system is obtained, such as, for example, in the manner described above wherein the microbial cells are derived from the $C_2$-$C_6$ alkyl compound utilizing microorganisms which have been aerobically grown in a nutrient medium containing the inducing growth substrate or enzyme preparations derived therefrom. The nutrient medium in which the microorganisms are induced and grown may be the culture medium described by Foster and Davis, J. Bacteriol., 91, 1924 (1966). Once the microorganisms have been induced and grown, the microbial cells are preferably harvested, washed and the resulting resting microbial cells or the resulting enzyme preparation may then be used as such to convert the substrates to the corresponding product(s) under aerobic conditions (in the presence of oxygen) in a buffered solution. The mixture of the substrate material and induced microbial cells or enzyme preparation in the buffered solution is incubated until the desired degree of conversion has been obtained. Thereafter, the product is recovered by conventional means, e.g., distillation, etc.

To facilitate the necessary effective contact of oxygen and the enzyme system (whether it be an enzyme preparation as such or microbial cells derived from the $C_2$-$C_6$ alkyl compound induced microorganisms), it is preferred, for best results, to employ a strong, finely divided air stream into a vigorously stirred dispersion of the substrate in the oxidation medium, which generally contains water and a buffer, and in which the enzyme preparation or induced microbial cell system is suspended. The enzyme preparation or induced microbial

cell system may then be separated from the liquid medium, preferably by filtration or centrifugation. The resulting product may then generally be obtained.

The process of the invention may be carried out batchwise, semicontinuously, continuously, concurrently or countercurrently. Optionally, the suspension containing the enzyme preparation or cells of microorganisms and buffer solution is passed downwardly with vigorous stirring countercurrently to an air stream rising in a tube reactor. The top layer is removed from the downflowing suspension, while culture and remaining buffer solution constituents are recycled, at least partly, with more oxidative substrate and addition of fresh enzyme preparation or induced microbial cell system, as required.

The growth of the microorganisms and the oxidation process may be conveniently coupled by conducting them simultaneously, but separately and using much higher aeration in the oxidation process (e.g., an air excess of at least twice that required for growth, preferably at least five times as much aeration). Both the growth process and the oxidative process may be conducted in the same reactor in sequential or simultaneous operations by alternate use of normal and strong aeration.

The invention is illustrated further by the following examples which, however, are not to be taken as limiting in any respect. All parts and percentages, unless expressly stated otherwise, are by weight.

EXAMPLE 1

A nutrient medium as described by Foster and

Davis, J. Bacteriol., 91, 1924 (1966), supra, having the following composition per liter of water was prepared:

| | |
|---|---|
| $Na_2HPO_4$ | 0.21 g |
| $NaH_2PO_4$ | 0.09 g |
| $NaNO_3$ | 2.0 g |
| $MgSO_4 \cdot 7H_2O$ | 0.2 g |
| KCl | 0.04 g |
| $CaCl_2$ | 0.015 g |
| $FeSO_4 \cdot 7H_2O$ | 1 mg |
| $CuSO_4 \cdot 5H_2O$ | 0.01 mg |
| $H_3BO_4$ | 0.02 mg |
| $MnSO_4 \cdot 5H_2O$ | 0.02 mg |
| $ZnSO_4$ | 0.14 mg |
| $MoO_3$ | 0.02 mg |

The pH of the nutrient medium was adjusted to 7.0 by the addition of acid or base, and 700 ml samples of the nutrient medium were charged into a plurality of 2.8 liter shake flasks. The shake flasks were inoculated with an inoculating loop of cells from an agar plate containing homogeneous colonies of the microorganisms on the plate (the purity of the isolates was confirmed by microscopic examination). The isolates had been maintained on agar plates under an atmosphere of ethane, propane, or butane and air having a 1:1 V/V gas ratio which had been transferred every two weeks. The gaseous phase of the inoculated flasks was replaced with a gas mixture comprised of ethane, propane or butane and air having a ratio of 1:1 on a V/V basis. The inoculated flasks were sealed air-tight and were incubated on a rotary shaker at 250 rpm and at 30°C for two days until turbidity in the medium had developed. The cells were harvested by centrifugation at 10,000 x g at 4°C for 30 minutes. The cell pellet was washed twice with a 0.05 M phosphate buffer at a pH of 7.0 (containing 0.005

M·$MgCl_2$). The washed cells were then suspended in a 0.05 M phosphate buffer at pH 7.0 to obtain an optical density at 660 nm of 0.5.

A 0.5 ml sample of each washed cell suspension (2 mg cells) was put into separate 10 ml vials at 4°C which were sealed with a rubber cap. The gaseous phase of the vials was removed with vacuum and then was replaced with a gas mixture of the substrate reactant (i.e., alkane or hydrocarbon) and oxygen at a 1:1 V/V ratio. (In the case of a liquid substrate, e.g., hexane, benzene and toluene, 10 µl of the substrate was put in the 10 ml vials). The vials were then incubated at 30°C on a rotary shaker at 300 rpm. Samples of product (1-3 µl) were withdrawn periodically with a microsyringe and the products were analyzed by gas chromatography (ionization flame detector column) at a column temperature of 100°C or 120°C and at a carrier gas flow rate of 30 or 35 ml/min of helium. The various products were identified by retention time comparisons and co-chromatography with authentic standards. The amount of product formed was determined from the peak area using a standard graph which had been constructed with an authentic standard. Protein in the cell suspensions was determined by the method described by O. H. Lowry et al., J. Biol. Chem., 193, 265 (1951).

EXAMPLE 2

Microbiological Conversion of Linear Alkanes to Methyl Ketones Using Propane-Grown Microorganism Strains

The procedure of Example 1 was followed to grow a plurality of microorganism strains identified in Table I under aerobic conditions in a nutrient medium containing propane as the growth substrate. The cells were harvested and washed as described in Example 1. The resting microbial cells thus obtained were then

contacted with $C_3$-$C_6$ n-alkanes in a buffered solution by the procedure of Example 1. The results of this series of experiments are shown in Table I.

EXAMPLE 3

Microbiological Conversion of Linear Alkanes to Methyl Ketones Using Ethane- and Butane-Grown Microorganism Strains

The procedure of Example 1 was followed for growth of three microorganism strains identified in Table II under aerobic conditions in a nutrient medium containing either ethane or butane as the growth substrate. The cells were harvested, washed and then contacted with $C_3$-$C_6$ n-alkanes in a buffered solution by the procedure of Example 1. The results of these oxidative conversions are given in Table II.

EXAMPLE 4

Microbiological Conversion of Linear Alkanes to Secondary Alcohols Using $C_2$-$C_4$ Alkane-Grown Microorganism Strains

Three of the microorganism strains from Example 2 identified in Table III, which were grown under aerobic conditions in a nutrient medium containing ethane, propane or butane, and harvested, washed and then contacted with the $C_3$-$C_6$ n-alkanes in a buffered solution by the procedure of Example 1, were analyzed for evidence of secondary alcohols as well as methyl ketones. The oxidation conversion rates for production of alcohols are indicated in Table III.

TABLE I

Microbiological Oxidation of Linear Alkanes to Methyl Ketones by Propane-Grown Strains

| Microorganism Strain Identification | Oxidation Conversion Rate ( μ moles/hr/mg protein) Propane to Acetone | Butane to 2-Butanone | Pentane to 2-Pentanone | Hexane to 2-Hexanone |
|---|---|---|---|---|
| *Arthrobacter petroleophagus* ATCC 21494 | 1.2 | 2.4 | 0.20 | 0.08 |
| *Arthrobacter simplex* ATCC 21032 | 0.8 | 1.6 | 0.06 | 0 |
| *Rhodococcus rhodochrous* (*Arthrobacter* sp.) ATCC 19140 | 1.1 | 1.8 | 0.06 | 0.02 |
| *Pseudomonas aeruginosa* (*Alcaligenes* sp.) ATCC 15525 | 1.3 | 1.8 | 0.20 | 0.09 |
| *Pseudomonas aeruginosa* (*Brevibacterium insectiphilum*) ATCC 15528 | 1.2 | 2.0 | ND | ND |
| *Brevibacterium* sp. ATCC 14649 | 1.2 | 1.9 | 0.08 | ND |
| *Brevibacterium fuscum* ATCC 15993 | 2.2 | 3.0 | 0.12 | 0.06 |

TABLE I (CONT'D)

| Microorganism Strain Identification | Oxidation Conversion Rate ( µ moles/hr/mg protein) Propane to Acetone | Butane to 2-Butanone | Pentane to 2-Pentanone | Hexane to 2-Hexanone |
|---|---|---|---|---|
| Alcaligenes eutrophus (Hydrogenomonas eutropha) ATCC 17697 | 1.0 | 1.2 | 0.30 | ND |
| Mycobacterium album ATCC 29676 | 1.4 | 2.0 | 0.20 | ND |
| Rhodococcus rhodochrous (Mycobacterium rhodochrous) ATCC 29670 | 1.3 | 1.9 | 0.40 | 0.10 |
| Rhodococcus rhodochrous (Mycobacterium rhodochrous) ATCC 29672 | 1.6 | 2.0 | ND | ND |
| Rhodococcus sp. (Nocardia neoopaca) ATCC 21499 | 1.6 | 2.8 | ND | ND |
| Rhodococcus rhodochrous (Nocardia paraffinica) ATCC 21198 | 2.0 | 4.4 | 0.60 | 0.01 |
| Pseudomonas crucurae NRRL B-1021 | 1.8 | 2.0 | ND | ND |

TABLE I (CONT'D)

| Microorganism Strain Identification | Oxidation Conversion Rate ( μ moles/hr/mg protein) Propane to Acetone | Butane to 2-Butanone | Pentane to 2-Pentanone | Hexane to 2-Hexanone |
|---|---|---|---|---|
| Pseudomonas fluorescens NRRL B-1244 | 1.0 | 1.2 | 0.08 | ND |
| Pseudomonas cepacia (Pseudomonas multivorans) ATCC 17616 | 1.0 | 1.1 | ND | ND |
| Pseudomonas putida Type A ATCC 17453 | 0.8 | ND | ND | ND |

ND = not determined

TABLE II

Microbiological Oxidation of Linear Alkanes to Methyl Ketones by Ethane- and Butane-Grown Strains

| Microorganism Strain Identification | Growth Substrate | Oxidation Conversion Rate ( μ moles/hr/mg protein) | | | |
|---|---|---|---|---|---|
| | | Propane to Acetone | Butane to 2-Butanone | Pentane to 2-Pentanone | Hexane to 2-Hexanone |
| *Brevibacterium fuscum* ATCC 15993 | ethane | 1.3 | 1.8 | 0.04 | 0.01 |
| *Brevibacterium fuscum* ATCC 15993 | butane | 1.7 | 2.0 | 0.02 | 0.01 |
| *Rhodococcus rhodochrous* (*Nocardia paraffinica*) ATCC 21198 | ethane | 1.2 | 1.6 | 0.4 | 0.01 |
| *Rhodococcus rhodochrous* (*Nocardia paraffinica*) ATCC 21198 | butane | 1.6 | 1.8 | 0.5 | 0.02 |
| *Rhodococcus rhodochrous* (*Mycobacterium rhodochrous*) ATCC 29670 | ethane | 1.2 | 1.7 | 0.38 | 0.10 |
| *Rhodococcus rhodochrous* (*Mycobacterium rhodochrous*) ATCC 29670 | butane | 1.2 | 1.8 | 0.41 | 0.09 |

TABLE III

Microbiological Oxidation of Linear Alkanes to Secondary Alcohols by $C_2$-$C_4$ Alkane-Grown Strains

| Microorganism Strain Identification | Growth Substrate | Oxidation Conversion Rate (μ moles/hr/mg protein) Propane to 2-Propanol | Butane to 2-Butanol | Pentane to 2-Pentanol | Hexane to 2-Hexanol |
|---|---|---|---|---|---|
| *Arthrobacter petroleophagus* ATCC 21494 | propane | 0.8 | 1.2 | 0.20 | 0.07 |
| *Rhodococcus rhodochrous* (*Mycobacterium rhodochrous*) ATCC 29670 | propane | 0.9 | 1.1 | 0.24 | 0.08 |
| *Pseudomonas aeruginosa* (*Alcaligenes* sp.) ATCC 15525 | propane | 1.8 | 1.6 | 0.41 | 0.18 |
| | ethane | 1.5 | 1.2 | 0.42 | 0.11 |
| | butane | 1.6 | 1.3 | 0.640 | 0.10 |

## EXAMPLE 5

## Microbiological Conversion of Saturated Alicyclic and Aromatic Hydrocarbons to Alcohols Using Alkane-Grown Microorganism Strains

The procedure of Example 1 was followed for growth of a plurality of microorganism strains identified in Table III under aerobic conditions in a nutrient medium containing ethane, propane, or butane as growth substrate. The cells were harvested, washed and then contacted with cyclohexane, benzene or toluene in a buffered solution by the procedure of Example 1, to yield secondary alcohols. The results of these oxidative conversions are shown in Table IV.

## EXAMPLE 6

## Optimal Conditions for Alkane Oxidation

The following summarizes tests conducted on the optimal conditions for the production of methyl ketones from alkanes. It will be understood that these were the optimal conditions found and that the invention is not to be bound thereby. Conversion can still be obtained by deviating from the optimum values indicated below, but with lower yields and conversions.

### Time Course

TABLE IV

Microbiological Oxidation of Saturated Alicyclic and Aromatic Hydrocarbons to Alcohols by $C_2$-$C_4$ Alkane-Grown Strains

| Microorganism Strain Identification | Growth Substrate | Oxidative Conversion Rate ( µ mole/hr/mg protein): Cyclohexane to Cyclohexanol | Benzene to Phenol | Toluene to Benzylalcohol |
|---|---|---|---|---|
| Arthrobacter simplex ATCC 21032 | propane | 0.37 | 0 | 0 |
| Pseudomonas aeruginosa (Alcaligenes sp.) ATCC 15525 | propane | 1.5 | 2.0 | 0 |
| Brevibacterium sp. ATCC 14649 | propane | 0 | 0.7 | 0 |
| Brevibacterium fuscum ATCC 15993 | ethane | 1.0 | 1.2 | 0 |
| | propane | 0 | 1.6 | 0 |
| | butane | 1.4 | 2.3 | 0.4 |
| Alcaligenes eutrophus (Hydrogenomonas eutropha) ATCC 17697 | propane | 0 | 1.9 | 0 |
| Rhodococcus rhodochrous (Mycobacterium rhodochrous) ATCC 29670 | propane | 1.3 | 1.7 | 0 |

TABLE IV (CONT'D)

| Microorganism Strain Identification | Growth Substrate | Oxidative Conversion Rate (µ mole/hr/mg protein) Cyclohexane to Cyclohexanol | Benzene to Phenol | Toluene to Benzylalcohol |
|---|---|---|---|---|
| Rhodococcus rhodochrous (Mycobacterium rhodochrous) ATCC 29672 | propane | 0 | 3.5 | 0 |
| Rhodococcus sp. (Nocardia neoopaca) ATCC 21499 | propane | 0 | 3.9 | 0 |
| Rhodococcus rhodochrous (Nocardia paraffinica) ATCC 21198 | ethane | 0.6 | 0.7 | 0 |
| | propane | 2.0 | 2.8 | 0 |
| | butane | 1.7 | 0 | 0 |
| Pseudomonas crucurae NRRL B-1021 | propane | 1.0 | 0.5 | 0 |
| Pseudomonas fluorescens NRRL B-1244 | propane | 0 | 1.4 | 0 |
| Pseudomonas aeruginosa (Pseudomonas ligustri) ATCC 15522 | propane | 0.7 | 0 | 0 |
| Pseudomonas putida Type A ATCC 17453 | propane | 0.37 | 1.7 | 0 |

The rate of production of methyl ketones from n-alkanes by cell suspensions of various $C_2$-$C_4$ alkane-grown microorganism strains was linear for the first hour of incubation. Therefore, the production of methyl ketone was measured within one hour of incubation whenever the effect of a variable was tested.

## pH

The effect of pH on the production of acetone or 2-butanone from propane or n-butane, respectively, using cell suspensions of propane-grown Rhodococcus rhodochrous (Nocardia paraffinica) ATCC 21198 was studied, wherein a 0.05M phosphate buffer was used for pH values from 5.5 to 8.0 and a 0.05M tris-(hydroxymethyl)aminomethane-HCl buffer for pH values from 8.0 to 10.0. The optimum pH for the production of acetone and 2-butanone was about 7.5.

## Temperature

The optimum temperature for the production of acetone or 2-butanone from propane or n-butane, respectively, using cell suspensions of propane-grown Rhodococcus rhodochrous (Nocardia paraffinica) ATCC 21198 was about 35°C.

## Substrate Specificity

From Tables I, II and III it can be seen that methyl ketones and secondary alcohols were produced from n-alkanes. Among the n-alkanes, n-propane and n-butane were oxidized at the highest rate.

From Table IV it is seen that alcohols were produced from cyclohexane, benzene and toluene for at least one strain tested.

## Product and Enzyme Analysis

All of the strains tested exhibited the ability to catalyze formation of methyl ketones. Some cultures, such as Rhodococcus rhodochrous (Nocardia paraffinica) ATCC 21198 and Brevibacterium fuscum ATCC 15993, showed greater activity for methyl ketone formation. Alkane-grown cells of at least three microorganism oxidized $C_3$ to $C_6$ alkanes to their corresponding 2-alcohols and methyl ketones.

In propane-grown cells, propane is metabolized through either terminal or subterminal oxidation. J. R. Vestal et al., J. Bacteriol., 99, 216 (1969), using Brevibacterium sp. JOB5, and E. R. Leadbetter et al., Arch. Mikrobiol., 35, 92 (1960), using M. smegmatis 422, suggested that propane was metabolized via subterminal oxidation. From the majority of propane-grown strains tested in the experiments herein 2-propanol and acetone were obtained from propane but not 1-propanol. To test if the propane monooxygenase system is a specific enzyme for subterminal oxidation or a nonspecific enzyme catalyzing both terminal and subterminal oxidation, so that 1-propanol was produced but could not be detected, the following experiment was performed.

1-Propanol, 2-propanol, or acetone in amounts of 0.3 to 0.4 μmoles/ml cell suspension was incubated with cell suspensions of both viable and heat-killed cells of propane-grown Rhodococcus rhodochrous (Nocardia paraffinica) ATCC 21198. The rates of disappearance of substrates were followed by gas-liquid chromatography. Suspensions of the heat-killed cells did not oxidize any of these substrates; however, with viable cells all of the 1-propanol added had disappeared (oxidized) within 6 min. of incubation. When the reaction mixture using

1-propanol as substrate was acidified, extracted with benzene and then methylated with $BF_3$-methanol, the presence of the methyl ester of propionic acid was confirmed by gas-liquid chromatography. 2-Propanol was also oxidized by the viable cell suspensions during the 90-min. incubation period. The 2-propanol is further oxidized to acetone, which accumulates, and the 1-propanol is further oxidized at a much faster rate to acid through aldehyde.

EXAMPLE 7

Cell-Free Soluble Fraction (alkane monooxygenase):

The Oxidation of Alkanes, Cyclic and Aromatic Compounds

The microorganism *Pseudomonas fluorescens* NRRL B-1244 was grown on propane (7% propane and 93% air) at 30°C in a batch culture on a mineral salt medium as described in Example 1 in a 30-liter fermentor (New Brunswick Scientific Co., Edison, N.J.). The fermentor was inoculated with 2 liters of a culture grown in flasks.

The cells thus grown were washed twice with 25 mM potassium phosphate buffer pH 7.0 and suspended in the same buffer solution containing 5mM $MgCl_2$ and deoxyribonuclease (0.05 mg/ml). Cell suspensions at 4°C were disintegrated by a single passage through a French pressure cell (American Instruments Co., Silver Spring, Md.) at 60 mPa. Disintegrated cell suspensions were centrifuged at 15,000 x g for 15 min. to remove unbroken cells. The supernatant solution was then centrifuged at 40,000 x g for 60 minutes and the supernatant solution therefrom was again centrifuged at 80,000 x g for 60 minutes, yielding the soluble fraction.

Several 3-ml vials at 4°C were charged with 0.2 ml of a reaction mixture consisting of 10 μ moles potassium phosphate buffer pH 7.0, 4 μ moles $NADH_2$, and the soluble enzyme fraction obtained above. The gaseous phase of the vials was evacuated by vacuum and replaced with a gas mixture of gaseous oxidation substrate and oxygen (1:1 v/v); in the case of a liquid oxidation substrate, 2 μl of substrate was added. The vials were incubated at 35°C on a reciprocating water bath shaker at 50 oscillations per minute.

The rate of epoxidation of alkenes and styrene was measured by injecting 1-2 μl samples of the reaction mixture into a gas chromatograph immediately after addition of substrate (zero time) and after 5 and 10 min. of incubation. Specific activities were expressed as μ moles of product formed per 10 min. per mg of protein. With each substrate, control experiments were conducted in the absence of $NADH_2$, in the absence of oxygen, and using boiled extracts. The results obtained are shown in Table V.

TABLE V

Oxidation of Alkanes, Cyclohexane and Toluene by Soluble Extracts of *Pseudomonas fluorescens* NRRL B-1244

| Oxidation Substrate | Product | Rate of Product Formation (μ mole/10 min./mg protein) |
|---|---|---|
| Ethane | Ethanol | 0.0070 |
| Propane | 1-Propanol | 0.0042 |
| | 2-Propanol | 0.0050 |
| Butane | 1-Butanol | 0.0070 |
| | 2-Butanol | 0.0072 |
| Isobutane | Isobutanol | 0.0046 |
| Pentane | 1-Pentanol | 0.0042 |
| | 2-Pentanol | 0.0046 |
| Hexane | 1-Hexanol | 0.0080 |
| | 2-Hexanol | 0.0021 |
| Cyclohexane | Cyclohexanol | 0.0050 |
| Toluene | Benzyl alcohol | 0.0025 |

## EXAMPLE 8

### Microbiological Conversion Of Alkenes And Dienes To 1,2-Epoxides Using Propane-Grown Microorganism Strains

The procedure of Example 1 was followed to grow a plurality of microorganism strains identified in Table VI under aerobic conditions in a nutrient medium containing propane as the growth substrate. The cells were harvested and washed as described in Example 1. The resting microbial cells thus obtained were then contacted with $C_2$-$C_6$ n-alkenes and butadiene in a buffered solution by the procedure of Example 1. The results of this series of experiments are shown in Table VI.

TABLE VI

MICROBIOLOGICAL OXIDATION OF ALKENES AND DIENES TO 1,2-EPOXIDES BY PROPANE-GROWN STRAINS

| Microorganism Strain Identification | Oxidative Conversion Rate ( μmole/hr/mg protein) | | | | | |
|---|---|---|---|---|---|---|
| | Ethylene to 1,2-Epoxyethane | Propylene to 1,2-Epoxypropane | 1-Butene to 1,2-Epoxybutane | 1,3-Butadiene to 1,2-Epoxybutene | 1-Pentene to 1,2-Epoxypentane | 1-Hexene to 1,2-Epoxyhexane |
| *Arthrobacter petroleophagus* ATCC 21494 | 0.41 | 0.32 | 0.36 | 0.59 | 0.10 | 0.06 |
| *Arthrobacter simplex* ATCC 21032 | 0.47 | 0.35 | 0.22 | 0.24 | 0.01 | 0 |
| *Rhodococcus rhodochrous* (*Arthrobacter* sp.) ATCC 19140 | 0.50 | 0.91 | 0.06 | 0.07 | 0.04 | 0 |
| *Pseudomonas aeruginosa* (*Alcaligenes* sp.) ATCC 15525 | 2.60 | 0.57 | 0.15 | 0.11 | 0.04 | 0.02 |
| *Pseudomonas aeruginosa* (*Brevibacterium insectiphilum*) ATCC 15528 | 0.1 | 0.3 | 0.1 | 0.05 | 0.03 | 0 |

TABLE VI (CONT'D)

| Microorganism Strain Identification | Ethylene to 1,2-Epoxy-ethane | Propylene to 1,2-Epoxy-propane | 1-Butene to 1,2-Epoxy-butane | 1,3-Butadiene to 1,2-Epoxy-butene | 1-Pentene to 1,2-Epoxy-pentane | 1-Hexene to 1,2-Epoxy-hexane |
|---|---|---|---|---|---|---|
| Brevibacterium sp. ATCC 14649 | 0.27 | 0.25 | 0.05 | 0.06 | 0.02 | 0.02 |
| Brevibacterium fuscum ATCC 15993 | 0.31 | 1.8 | 0.63 | 0.34 | 0.12 | 0.06 |
| Alcaligenes eutrophus (Hydrogenomonas eutropha) ATCC 17697 | 0.40 | 0.40 | 0.08 | 0.02 | 0.01 | 0 |
| Mycobacterium album ATCC 29676 | 0.36 | 0.36 | 0.16 | 0.08 | 0.05 | 0.01 |
| Mycobacterium paraffinicum ATCC 12670 | 0.10 | 0.11 | 0.04 | 0.01 | 0.01 | 0 |
| Rhodococcus rhodochrous (Mycobacterium rhodochrous) ATCC 29670 | 0.30 | 0.20 | 0.15 | 0.08 | 0 | 0 |

TABLE VI (CONT'D)

| Microorganism Strain Identification | Ethylene to 1,2-Epoxy-ethane | Propylene to 1,2-Epoxy-propane | 1-Butene to 1,2-Epoxy-butane | 1,3-Butadiene to 1,2-Epoxy-butene | 1-Pentene to 1,2-Epoxy-pentane | 1-Hexene to 1,2-Epoxy-hexane |
|---|---|---|---|---|---|---|
| Rhodococcus rhodochrous (Mycobacterium rhodochrous) ATCC 29672 | 0.30 | 0.40 | 0.07 | 0.06 | 0.02 | 0.02 |
| Rhodococcus rhodochrous (Mycobacterium rhodochrous) ATCC 184 | 1.4 | 0.9 | ND | ND | ND | ND |
| Rhodococcus sp. (Nocardia neoopaca) ATCC 21499 | 0.07 | 0.37 | 0.68 | 0.70 | 0.03 | 0.02 |
| Pseudomonas crucurae NRRL B-1021 | 0.57 | 0.60 | 0.89 | 0.66 | 0 | 0 |
| Pseudomonas fluorescens NRRL B-1244 | 1.70 | 6.10 | 0.82 | 0.33 | 0.07 | 0.03 |

TABLE VI (CONT'D)

| Microorganism Strain Identification | Ethylene to 1,2-Epoxy-ethane | Propylene to 1,2-Epoxy-propane | 1-Butene to 1,2-Epoxy-butane | 1,3-Butadiene to 1,2-Epoxy-butene | 1-Pentene to 1,2-Epoxy-pentane | 1-Hexene to 1,2-Epoxy-hexane |
|---|---|---|---|---|---|---|
| Pseudomonas cepacia (Pseudomonas multivorans) ATCC 17616 | 0.40 | 0.79 | 0.38 | 0.19 | 0.05 | 0.03 |
| Pseudomonas putida Type A ATCC 17453 | 0.44 | 1.90 | 0.68 | 0.77 | 0.05 | 0.01 |
| Pseudomonas aeruginosa (Pseudomonas ligustri) ATCC 15522 | 0.46 | 0.48 | 0.34 | 0.69 | 0.04 | 0.02 |

ND = Not determined

## EXAMPLE 9

### Microbiological Conversion Of Alkenes And Dienes To 1,2-Epoxides Using Ethane- And Butane-Grown Microorganism Strains

The procedure of Example 1 was followed for growth of two microorganism strains identified in Table VII under aerobic conditions in a nutrient medium containing either ethane or butane as the growth substrate. The cells were harvested, washed and then contacted with $C_2$-$C_5$ n-alkenes and butadiene in a buffered solution by the procedure of Example 1. The *Mycobacterium* strain was only tested for conversion of isobutene. The results of these oxidative conversions are given in Table VII.

TABLE VII

MICROBIOLOGICAL OXIDATION OF ALKENES AND DIENES TO 1,2-EPOXIDES BY ETHANE- AND BUTANE-GROWN STRAINS

| Microorganism Strain Identification | Growth Substrate | (Oxidative Conversion Rate (μ Mole/hr/mg protein)) Ethylene to 1,2-Epoxyethane | Propylene to 1,2-Epoxypropane | 1-Butene to 1,2-Epoxybutane | Isobutene to 1,2-Epoxyisobutene | 1,3-Butadiene to 1,2-Epoxybutane | 1-Pentene to 1,2-Epoxypentane |
|---|---|---|---|---|---|---|---|
| *Brevibacterium fuscum* ATCC 15993 | ethane | 1.05 | 1.60 | 0.14 | ND | 0.07 | 0 |
| *Brevibacterium fuscum* ATCC 15993 | butane | 0.5 | 1.0 | 0.1 | ND | 0.2 | 0.01 |
| *Rhodococcus rhodochrous* (*Mycobacterium rhodochrous*) ATCC 29670 | ethane | ND | ND | ND | 0.06 | ND | ND |

ND = Not determined

EXAMPLE 10

Optimal Conditions For Epoxidation

The following summarizes tests conducted on the optimal conditions for the production of 1,2-epoxypropane from propylene, which is a preferred substrate. It will be understood that these were the optimal conditions found and that the invention is not to be bound thereby. Conversions can still be obtained by deviating from the optimum values indicated below, but with lower yields and conversions.

## Time Course

The production of propylene oxide from propylene reached a maximum after 2 to 3 hours of incubation in batch experiments for all the microorganism strains tested. The amount of propylene oxide did not decline after 20 hours of incubation. The rate of propylene oxide production was linear for the first 60 minutes. Therefore, the production of propylene oxide was measured within one hour of incubation whenever the effect of a variable was tested.

## pH

The effect of pH on the production of propylene oxide was studied using 0.05 M sodium phosphate buffer for pH values from 5.5 to 8.0 and using 0.05 M tris-(hydroxymethyl)aminomethane-HCl buffer for pH values of 8.0 to 10.0. The optimum pH for the production of propylene oxide was found to be in the range from 6.0 to 7.0 for all resting cell suspensions of the strains tested.

## Temperature

The optimum temperature for the propylene oxide production by cell suspensions was about 35°C for all strains tested.

## Product and Conversion Analysis

n-Propanol-grown cells showed no activity for epoxidation of propylene. Propane-grown cells in the form of resting cell suspensions yielded propylene oxide, which accumulated extracellularly. Control experiments with heat-killed cells indicated that the epoxide was produced enzymatically. No product peak other than propylene oxide was detected. Although S.W. May et al, J. Biol. Chem., 248, 1725 (1973) reported epoxide accumulation from 1-octene by *Pseudomonas aeruginosa* cells was accompanied by metabolism of a large amount of 1-octene via methyl group oxidation, the epoxidation of propylene by cell suspensions of propane-grown bacterial cells herein yielded no production of 3-hydroxy-1-propene.

## Substrate Specificity

From Table I it can be seen that epoxides were produced from both gaseous 1-alkenes and liquid alkenes as well as butadiene. The highest production of epoxides was found using ethylene or propylene as substrate for all propane-grown strains tested.

Substrate specificity for epoxidation of 1-alkenes and butadiene by various ethane- and butane-grown bacterial cells was also studied (see Table VII). Both strains tested oxidized 1-alkenes and butadiene to their corresponding 1,2-epoxides, with the preferred substrate being propylene.

EXAMPLE 11

Cell-Free Soluble Fraction (alkane monooxygenase): The Epoxidation of Alkenes and Styrene

The microorganism *Pseudomonas fluorescens* NRRL B-1244 was grown on propane (7% propane and 93% air) at 30°C in a batch culture on a mineral salt medium as described in Example 1 in a 30-liter fermentor (New Brunswick Scientific Co., Edison, N.J.). The fermentor was inoculated with 2 liters of a culture grown in flasks.

The cells thus grown were washed twice with 25 mM potassium phosphate buffer pH 7.0 and suspended in the same buffer solution containing 5mM $MgCl_2$ and deoxyribonuclease (0.05 mg/ml). Cell suspensions at 4°C were disintegrated by a single passage through a French pressure cell (American Instruments Co., Silver Spring, Md.) at 60 mPa. Disintegrated cell suspensions were centrifuged at 15,000 x g for 15 min. to remove unbroken cells. The supernatant solution was then centrifuged at 40,000 x g for 60 minutes and the supernatant solution therefrom was again centrifuged at 80,000 x g for 60 minutes, yielding the soluble fraction.

Several 3-ml vials at 4°C were charged with 0.2 ml of a reaction mixture consisting of 10 μ moles potassium phosphate buffer pH 7.0, 4 μmoles $NADH_2$, and the soluble enzyme fraction obtained above. The gaseous phase of the vials was evacuated by vacuum and replaced with a gas mixture of gaseous oxidation substrate and oxygen (1:1 v/v); in the case of a liquid oxidation substrate, 2 μl of substrate was added. The vials were incubated at 35°C on a reciprocating water bath shaker at 50 oscillations per minute.

The rate of epoxidation of alkenes and styrene was measured by injecting 1-2 μl samples of the reaction mixture into a gas chromatograph immediately after addition of substrate (zero time) and after 5 and 10 min. of incubation. Specific activities were expressed as moles of product formed per 10 min. per mg of protein. With each substrate, control experiments were conducted in the absence of $NADH_2$, in the absence of oxygen, and using boiled extracts. The results obtained are shown in Table VIII.

TABLE VIII

Epoxidation of Alkenes and Styrene by Soluble Extracts of *Pseudomonas fluorescens* NRRL B-1244

| Oxidation Substrate | Product | Rate of Product Formation (μ mole/10 min./mg protein) |
|---|---|---|
| Ethylene | Ethylene oxide | 0.025 |
| Propylene | Propylene oxide | 0.036 |
| 1-Butene | 1,2-Epoxybutane | 0.020 |
| 1,3-Butadiene | 1,2-Epoxybutene | 0.026 |
| Isobutene | Epoxyisobutane | 0.030 |
| *cis*-But-2-ene | *cis*-2,3-Epoxybutane | 0.016 |
| *trans*-But-2-ene | *trans*-2,3-Epoxybutane | 0.018 |
| 1-Pentene | 1,2-Epoxypentane | 0.017 |
| 1-Hexene | 1,2-Epoxyhexane | 0.016 |
| 1-Heptene | 1,2-Epoxyheptane | 0.012 |
| Styrene | Styrene oxide | 0.003 |

## EXAMPLE 12

### Regeneration of Electron Carrier

It has been found that the monooxygenase which catalyzes the epoxidation reaction requires an electron carrier (cofactor) such as NADH or NADPH for

its activity. When the cofactor is depleted, it can be regenerated by the addition of compounds which are substrates for dehydrogenases or oxidases such as alcohol (primary and/or secondary) dehydrogenase, aldehyde dehydrogenase, formate dehydrogenase, steroid dehydrogenase, etc. in either cell-free or whole cell systems. The overall catalyst system is stabilized by coupling this cofactor regeneration system to the epoxidation reaction process.

A schematic explanation of the coupling cycle for the regeneration of cofactor is shown below:

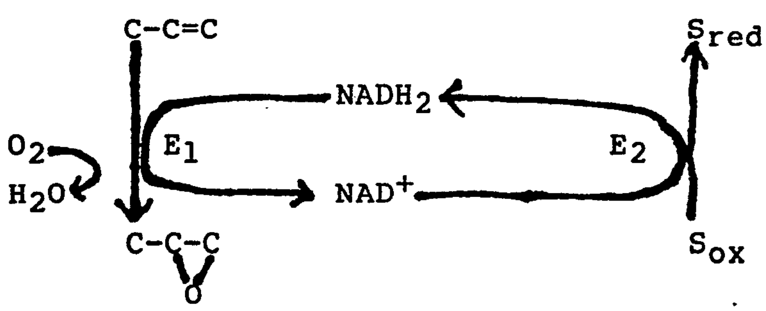


wherein $E_1$ is the monooxygenase enzyme, $E_2$ is the dehydrogenase enzyme and S is the substrate for cofactor regeneration.

<u>Cofactor regeneration through ethanol dehydrogenase</u>

Two microorganism strains indicated in Table IX were grown on propane and used to epoxidize propylene to propylene oxide as described in Example 1, except that varying amounts of ethanol were added to the reaction mixtures in separate experiments, ethanol being a cofactor regeneration substrate. Ethanol was converted to acetaldehyde (acetyl Co~A in vivo) by primary alcohol dehydrogenase, yielding 1 mole of NADH, with subsequent oxidation of the intermediate acetyl Co~A to carbon dioxide, yielding 3 additional moles of

NADH. The results, indicated in Table IX, show that the epoxidation rate was improved on addition of 5 μ mole ethanol. Some inhibition was observed at higher ethanol concentration (25 μmoles) within the first two hours of reaction.

TABLE IX

| Microorganism Strain Identification | Epoxidation Rate (rate of propylene oxide formation in μ mole/0.5 ml) | | |
|---|---|---|---|
| | 1 hr. | 2 hr. | 3 hr. |
| *Pseudomonas fluorescens* NRRL B-1244 | | | |
| Control (no ethanol) | 4.0 | 6.0 | 6.2 |
| +5 μ mole ethanol | 4.6 | 8.2 | 9.5 |
| +25 μ mole ethanol | 3.8 | 8.0 | 9.6 |
| *Brevibacterium fuscum* ATCC 15993 | | | |
| Control (no ethanol) | 1.8 | 3.1 | 3.2 |
| +5 μ mole ethanol | 2.3 | 4.5 | 5.5 |
| +25 μ mole ethanol | 2.0 | 4.4 | 5.6 |

## EXAMPLE 13

### Microbiological Conversion of Linear Secondary Alcohols to Methyl Ketones Using Alkane-Grown Microorganism Strains

The procedure of Example 1 was followed to grow a plurality of microorganism strains identified in Table X under aerobic conditions in a nutrient medium containing ethane, butane or propane as the growth substrate, primarily propane. The cells were harvested and washed as described in Example 1. The resting microbial cells thus obtained were then contacted with $C_3$-$C_7$ linear secondary alcohols in a buffered

solution by the procedure of Example 1. The results of this series of experiments are shown in Table X.

EXAMPLE 14

Microbiological Conversion of Linear Secondary Alcohols to Methyl Ketones Using Alcohol or Alkylamine-Grown Microorganism Strains

The procedure of Example 1 was followed for growth of two microorganism strains identified in Table XI under aerobic conditions in a nutrient medium containing ethanol, propanol, butanol, ethylamine, propylamine or butylamine as the growth substrate. The cells were harvested, washed and then contacted with $C_3$-$C_7$ secondary alcohols in a buffered solution by the procedure of Example 1. The results of these oxidative conversions are given in Table XI.

Table X

MICROBIOLOGICAL OXIDATION OF LINEAR SEC. ALCOHOLS TO METHYL KETONES BY $C_2$-$C_4$-ALKANE-GROWN STRAINS

| Microorganism Strain Identification | Growth Substrate | Oxidative Conversion Rate (μ mole/hr/mg protein): 2-Propanol to Acetone | 2-Butanol to 2-Butanone | 2-Pentanol to 2-Pentanone | 2-Hexanol to 2-Hexanone | 2-Heptanol to 2-Heptanone |
|---|---|---|---|---|---|---|
| Arthrobacter petroleophagus ATCC 21494 | propane | 10.3 | 10.7 | 0.20 | 0.08 | 0.05 |
| Arthrobacter simplex ATCC 21032 | propane | 6.8 | 6.7 | 0.19 | 0.02 | 0.01 |
| Rhodococcus rhodochrous (Arthrobacter sp.) ATCC 19140 | propane | 8.4 | 6.7 | 0.88 | 0.05 | 0.01 |
| Pseudomonas aeruginosa (Alcaligenes sp.) ATCC 15525 | propane | 14.4 | 7.3 | 1.5 | 0.22 | 0 |
| Pseudomonas aeruginosa (Brevibacterium insectiphilum) ATCC 15528 | propane | 11.5 | 9.3 | 1.6 | 0.08 | 0.01 |
| Brevibacterium sp. ATCC 14649 | propane | 9.8 | 8.8 | 0.97 | 0.06 | 0.01 |
| Brevibacterium fuscum ATCC 15993 | ethane | 7.8 | 8.4 | 1.1 | 0.05 | 0.01 |
| | propane | 18.8 | 18.2 | 1.5 | 0.01 | 0 |
| | butane | 8.0 | 9.5 | 0.53 | 0.05 | 0.01 |

Table X Continued

| Microorganism Strain Identification | Growth Substrate | Oxidative Conversion Rate ($\mu$ mole/hr/mg protein): 2-Propanol to Acetone | 2-Butanol to 2-Butanone | 2-Pentanol to 2-Pentanone | 2-Hexanol to 2-Hexanone | 2-Heptanol to 2-Heptanone |
|---|---|---|---|---|---|---|
| Alcaligenes eutrophus (Hydrogenomonas eutropha) ATCC 17697 | propane | 8.2 | 8.0 | 1.2 | 0.09 | 0 |
| Mycobacterium album ATCC 29676 | propane | 10.8 | 9.2 | 1.2 | 0.11 | 0 |
| Mycobacterium paraffinicum ATCC 12670 | propane | 7.1 | 10.4 | 0.90 | 0.07 | 0.01 |
| Rhodococcus rhodochrous (Mycobacterium rhodochrous) ATCC 29670 | propane | 6.7 | 6.9 | 0.72 | 0.06 | 0 |
| Rhodococcus rhodochrous (Mycobacterium rhodochrous) ATCC 29672 | propane | 10.3 | 9.9 | 0.87 | 0.44 | 0 |
| Rhodococcus sp. (Nocardia neoopaca) ATCC 21499 | propane | 13.8 | 14.9 | 0.04 | 0.01 | 0.01 |
| Rhodococcus rhodochrous (Nocardia paraffinica) ATCC 21198 | ethane | 5.8 | 6.0 | 0.51 | 0.04 | 0 |
| | propane | 15.4 | 16.8 | 1.3 | 0.02 | 0.01 |
| | butane | 7.5 | 7.8 | 1.0 | 0.13 | 0.01 |

Table X Continued

| Microorganism Strain Identification | Growth Substrate | Oxidative Conversion Rate ($\mu$ mole/hr/mg protein) 2-Propanol to Acetone | 2-Butanol to 2-Butanone | 2-Pentanol to 2-Pentanone | 2-Hexanol to 2-Hexanone | 2-Heptanol to 2-Heptanone |
|---|---|---|---|---|---|---|
| Pseudomonas crucurae NRRL B-1021 | propane | 10.8 | 12.5 | 1.8 | 0.10 | 0.02 |
| Pseudomonas fluorescens NRRL B-1244 | propane | 12.9 | 15.3 | 2.4 | 0.13 | 0 |
| Pseudomonas cepacia (Pseudomonas multivorans) ATCC 17616 | propane | 12.8 | 11.4 | 0.07 | 0.01 | 0.01 |
| Pseudomonas putida Type A ATCC 17453 | propane | 9.3 | 10.3 | 0.89 | 0.08 | 0.02 |
| Pseudomonas aeruginosa (Pseudomonas ligustri) ATCC 15522 | propane | 7.7 | 15.4 | 1.8 | 0.18 | 0 |

Table XI

MICROBIOLOGICAL OXIDATION OF LINEAR SEC. ALCOHOLS TO METHYL KETONES BY STRAINS GROWN ON $C_2$-$C_4$ ALCOHOLS OR ALKYLAMINES

| Microorganism Strain Identification | Growth Substrate | Oxidative Conversion Rate (µ mole/hr/mg protein) | | | | |
|---|---|---|---|---|---|---|
| | | 2-Propanol to Acetone | 2-Butanol to 2-Butanone | 2-Pentanol to 2-Pentanone | 2-Hexanol to 2-Hexanone | 2-Heptanol to 2-Heptanone |
| Brevibacterium fuscum ATCC 15993 | ethanol | 10.2 | 12.7 | 0.7 | 0.01 | 0.005 |
| | n-propanol | 11.3 | 14.8 | 1.1 | 0.01 | 0.006 |
| Brevibacterium fuscum ATCC 15993 | ethylamine | 7.4 | 8.6 | 0.7 | 0 | 0 |
| | propylamine | 8.2 | 10.3 | 0.8 | 0 | 0 |
| | butylamine | 6.8 | 7.7 | 0.7 | 0 | 0 |
| Rhodococcus rhodochrous (Nocardia paraffinica) ATCC 21198 | ethanol | 7.0 | 10.5 | 0.2 | 0.01 | 0.004 |
| | n-propanol | 9.4 | 12.0 | 0.8 | 0.02 | 0.01 |
| Rhodococcus rhodochrous (Nocardia paraffinica) ATCC 21198 | ethylamine | 6.5 | 8.0 | 0.4 | 0 | 0 |
| | propylamine | 7.3 | 9.7 | 0.6 | 0 | 0 |
| | butylamine | 5.9 | 6.6 | 0.2 | 0 | 0 |

EXAMPLE 15

Optimal Conditions For Alcohol Oxidation

The following summarizes tests conducted on the optimal conditions for the production of methyl ketones from secondary alcohols. It will be understood that these were the optimal conditions found and that the invention is not to be bound thereby. Conversions can still be obtained by deviating from the optimum values indicated below, but with lower yields and conversions.

Time Course

The rate of production of methyl ketones from secondary alcohols by cell suspension of various $C_2$-$C_4$ alkane-grown microorganism strains was linear for the first three hours of incubation. Therefore, the production of methyl ketone was measured within three hours of incubation whenever the effect of a variable was tested.

pH

The effect of pH on the production of acetone or 2-butanone from 2-propanol or 2-butanol, respectively, using cell suspensions of propane-grown Rhodococcus rhodochrous (Nocardia paraffinica) ATCC 21198 was studied, wherein a 0.05 M phosphate buffer was used for pH values from 5.5 to 8.0 and a 0.05 M tris-(hydroxymethyl)-aminomethane-HCl buffer for pH values from 8.0 to 10.0. The optimum pH for the production of acetone and 2-butanone was in the range of about 8.0 to 9.0.

Temperature

The optimum temperature for the production of acetone or 2-butanone from 2-propanol or 2-butanol,

respectively, using cell suspensions of propane-grown Rhodococcus rhodochrous (Nocardia paraffinica) ATCC 21198 was about 35°C.

## Substrate Specificity

From Table X it can be seen that methyl ketones were produced from secondary alcohols using alkane-grown cells. From Table XI it can be seen that methyl ketones were produced from secondary alcohols using alcohol or alkylamine-grown cells. Among the secondary alcohols, 2-propanol and 2-butanol were oxidized at the highest rate. Some cultures, such as Rhodococcus rhodochrous (Nocardia paraffinica) ATCC 21198 and Brevibacterium fuscum ATCC 15993, showed greater activity for methyl ketone formation.

## Enzyme Analysis

It was reported by C. T. Hou et al., App. Environ. Microbiol., 38, 135 (1979) that a secondary alcohol specific dehydrogenase (SADH) catalyzed the oxidation of 2-alcohols to their corresponding methyl ketones using methylotrophic bacteria and methanol-grown yeast. In the present invention, SADH activity was found in the propane-grown and 1-propanol-grown cells of the microorganisms.

In summary, the present invention is seen to provide a process for oxidation of several hydrocarbons by contacting them under aerobic conditions with cells derived from selected microorganisms or enzyme preparations thereof which were previously grown aerobically in the presence of a $C_2$-$C_6$ alkane or, if the hydrocarbon is a secondary alcohol, also a $C_2$-$C_6$ alkyl radical donating compound such as an alcohol or alkylamine.

CLAIMS:

1. A process for oxidizing a substrate selected from alkanes, saturated alicyclic or aromatic hydrocarbons, alkenes, dienes, vinyl aromatic compounds, or secondary alcohols to the corresponding oxidation product or products by contacting the substrate in a reaction medium under aerobic conditions with microbial cells derived from a microorganism, a genetically engineered derivative or natural mutant of the microorganism, or an enzyme preparation prepared from the cells, which microorganism, derivative, mutant or preparation exhibits oxygenase or dehydrogenase enzyme activity, until the corresponding product or products are produced in at least isolatable amounts,

in which the microorganism has been previously grown under aerobic conditions in a nutrient medium containing a compound which is a $C_2$-$C_6$ alkane or, if the substrate is an alcohol, the compound may additionally be a $C_2$-$C_6$ alkyl radical donating compound, and in which the microorganism strains are selected from

Rhodococcus rhodochrous
(Arthrobacter sp.) ATCC 19140

Pseudomonas aeruginosa
(Alcaligenes sp.) ATCC 15525

Arthrobacter petroleophagus ATCC 21494

Arthrobacter simplex ATCC 21032

Pseudomonas aeruginosa
(Brevibacterium insectiphilum) ATCC 15528

Brevibacterium sp. ATCC 14649

Brevibacterium fuscum ATCC 15993

Alcaligenes eutrophus
(Hydrogenomonas eutropha) ATCC 17697

Mycobacterium album ATCC 29676

Rhodococcus rhodochrous
(Mycobacterium rhodochrous) ATCC 29670

*Rhodococcus rhodochrous* (*Mycobacterium rhodochrous*) ATCC 29672

*Rhodococcus* sp. (*Nocardia neoopaca*) ATCC 21499

*Pseudomonas crucurae* NRRL B-1021

*Pseudomonas fluorescens* NRRL B-1244

*Pseudomonas cepacia* (*Pseudomonas multivorans*) ATCC 17616

*Pseudomonas putida* Type A ATCC 17453, and

*Pseudomonas aeruginosa* (*Pseudomonas ligustri*) ATCC 15522,

wherein further if the substrate is an alkene, diene or vinyl aromatic compound, the microorganism strains may additionally be selected from

*Mycobacterium paraffinicum* ATCC 12670, and

*Rhodococcus rhodochrous* (*Mycobacterium rhodochrous*) ATCC 184,

and wherein further if the substrate is a secondary alcohol, the microorganism strains may additionally be selected from

*Mycobacterium paraffinicum* ATCC 12670, and

*Rhodococcus rhodochrous* (*Nocardia paraffinica*) ATCC 21198,

and wherein further if the substrate is an alkene or saturated alicyclic or aromatic hydrocarbon, the microorganism strain may additionally be:

*Rhodococcus rhodochrous* (*Nocardia paraffinica*) ATCC 21198.

2. A process according to claim 1 in which the $C_2$-$C_6$ alkane is a $C_2$-$C_4$ alkane.

3. A process according to claim 1 or 2 in which the strain is a Pseudomonas aeruginosa strain.

4. A process according to any one of claims 1 to 3 in which the substrate is a $C_2$-$C_6$ alkane, cyclohexane, benzene, $C_2$-$C_7$ alkene, butadiene, styrene or a $C_3$-$C_6$ secondary alcohol.

5. A process according to any one of claims 1 to 3 in which the substrate is an alkene, diene or vinyl aromatic compound and an electron carrier or other biochemical species which drives the oxidation is present in the reaction medium.

6. A process according to claim 5 in which the electron carrier is NADH and is regenerated.

7. A process according to any one of claims 1 to 4 in which the microbial cells are resting cells.

8. A process according to any one of claims 1 to 4 in which the microbial cells are in the form of a resting cell suspension.

9. A process according to any one of claims 1 to 4 in which the enzyme preparation is a cell-free soluble fraction.

10. A process according to any one of the preceding claims in which the $C_2$-$C_6$ alkyl radical donating compound is ethanol, propanol, butanol, ethylamine, propylamine or butylamine and the substrate is a $C_3$-$C_5$ secondary alcohol.